# EUROPEAN PATENT APPLICATION

(11) **EP 4 591 778 A1**
(43) Date of publication of application: **30.07.2025**
(21) Application number: 24305141.4
(22) Date of filing: 26.01.2024
(51) Int. Cl.: A61B 1/00, A61B 6/00

(54) **SYSTEM FOR TESTING AND IMAGE ACQUISITION OF A MEDICAL INJECTION DEVICE**

(71) Applicant: Becton Dickinson France, 38800 Le Pont-de-Claix (FR)
(72) Inventor: FUENTES, Cynthia, 38450 VIF (FR); GAGLIANO, Julien, 38240 MEYLAN (FR)
(74) Representative: Germain Maureau

(57) **Abstract**

Provided herein is a system for testing of a medical injection device. The system includes a testing apparatus for testing the device, with the testing apparatus including a frame having a bottom base, a top support spaced configured to secure a first portion of the injection device, and side track members. Guide rails extend vertically between the top support and the bottom base and a holder is movable along the guide rails and the side track members, with the holder securing a second portion of the injection device. An actuator is coupled to the frame and configured to move the holder along the guide rails, with movement of the holder causing a relative movement between the second and first portions of the injection device. An x-ray imaging system acquires x-ray images of the injection device, to capture the relative movement between the second and first portions of the device.

## Description

### BACKGROUND OF THE INVENTION

### Field of the Invention

The present disclosure relates generally to syringes and, more particularly, to syringe systems employed for the manufacture of syringe barrels having a baked silicone coating thereon, with the systems supporting the syringe barrels to prevent or minimize weakening of the syringe barrel tip that can occur during baking thereof.

### Description of Related Art

Syringes are used in a variety of environments for administering fluids, such as medications or other drugs, to a patient. A syringe will typically include a glass (or plastic) syringe barrel with an opening at a distal end and a plunger assembly inserted through the opposite proximal end of the barrel. The distal end of the syringe barrel may include a tip through which the opening is formed. In some embodiments, the tip may be configured as a luer connection that enables a transfer of fluid out from or into the syringe barrel, while in other embodiments the tip may be configured as a hub that retains a needle therein for the syringe. The plunger assembly typically includes an elongated plunger rod extending out of the barrel and a plunger head or stopper disposed at the distal end of the plunger rod. The stopper is typically made of elastomeric material and is adapted to ensure the container closure integrity of a syringe when the stopper is inserted into the syringe.

The plunger assembly is movable within the syringe barrel to force fluid out from an inner volume of the syringe barrel and/or to aspirate fluid into the inner volume of the syringe barrel - with the stopper sliding through the barrel when the plunger assembly is actuated by a user. In order to improve sliding of the stopper within the barrel, it is known in the art to lubricate the barrel with a lubricant coating, such as a silicone coating as a primary example. The coating can be deposited on the inner walls of the barrel by spraying, dipping or another suitable application technique. The siliconization of the inner wall of the syringe barrel allows a smooth movement or gliding of the stopper within the chamber.

As known in the art, a rigid tip cap or needle shield is typically included on the syringe to maintain sterility of the syringe tip and, where the tip includes a needle thereon, to provide protection against accidental needle sticks. The tip cap is removable from the syringe via application of a pulling force thereto (i.e., a "pull-out force"), with it being desirable for the tip cap to be removable in a predictable and consistent manner. In some known syringes, tip caps are designed to include frangible features that may be broken during removal thereof, with such frangible features providing evidence of tampering or prior use of the syringe.

With regard to syringes as described above, it is desirable to provide for testing of such syringes and, in particular, to provide for testing regarding the interaction of components of the syringe during use thereof. As primary examples, it is desirable to test and analyze how the stopper moves within the syringe barrel during actuation/movement of the plunger assembly (e.g., deformation of the stopper, characteristics/reaction of the lubricant layer, etc.) and/or how the tip cap reacts in being pulled off of the syringe (e.g., deformation of the tip cap and/or breaking of a frangible feature). This testing of the syringe may include acquiring a series of images of the syringe during movement and/or actuation of components thereof, which may include the capturing of video images and/or x-ray images (or images at other wavelengths) of the syringe.

The testing and associated image capture for syringes as described above may be accomplished via the use of dedicated tooling. The tooling may be designed to ensure stability of the syringe during testing (that might disturb/distort acquired images) and allow for the acquisition of images of the syringe without interference from features/components of the tooling. However, it is recognized that certain constraints may be placed on the tooling depending on the testing and analysis being performed. As an example, where it is desired to perform syringe testing and acquire associated x-ray images of the syringe during testing, constraints may be placed on the tooling regarding the materials used therein and the protections provided therewith that allow for the use of an x-ray imaging device in the testing. Such constraints may lead to challenges as to how to design and operate the tooling.

Accordingly, a need exists in the art for tooling that provides for image acquisition of a syringe during testing, including x-ray images and/or other video images. The tooling should provide for remote operation thereof, such as may be involved with actuating the syringe during testing. The tooling should allow be multi-functional, so as to enable testing of various types/sizes of syringe, and for the testing of different components of the syringe, such as may be desired for testing associated with movement of the plunger assembly and/or removal of a tip cap.

### SUMMARY OF THE INVENTION

Provided herein is a system for testing of a medical injection device. The system includes a testing apparatus configured to retain the medical injection device for testing thereof, with the testing apparatus including an open frame comprising a bottom base, a top support spaced vertically from the bottom base and configured to secure a first portion of the medical injection device, and side track members extending between the top support and the bottom base. The testing apparatus also includes guide rails extending vertically between the top support and the bottom base, a holder movable along the guide rails and the side track members relative to the frame and configured to secure a second portion of the medical injection device, and an actuator coupled to the frame and configured to move the holder in an upward direction and a downward direction along the guide rails, with movement of the holder causing a relative movement between the second portion and the first portion of the medical injection device. The system also includes an x-ray imaging system configured to acquire x-ray images of the medical injection device, so as to capture the relative movement between the second portion and the first portion of the medical injection device.

In certain configurations, the system further includes an x-ray proof enclosure positioned about the testing apparatus and the x-ray imaging system.

In certain configurations, the system further includes a loading device configured to provide the medical injection device to the testing apparatus, with the loading device transferring the medical injection device to the holder.

In certain configurations, the system further includes a remote control configured to control operation of the actuator.

In certain configurations, the remote control includes a first control input to control movement of the holder in the upward direction and the downward direction and a second control input to control a speed of the holder as it moves along the guide rails.

In certain configurations, the actuator comprises an outer sleeve, an extension rod movable within the outer sleeve, with a bottom end of the extension rod joined to the holder, and a servo motor configured to linearly translate the extension rod into and out from the outer sleeve, to move the holder in the upward direction and the downward direction.

In certain configurations, the testing apparatus comprises a pair of stops positioned on the side track members and configured to limit a range of movement of the holder in the upward and downward directions along the side track members.

In certain configurations, the top support includes a retention slot formed therein by which the first portion of the medical injection device is secured to the top support.

In certain configurations, the testing apparatus comprises a fluid collector positioned on the bottom base, the fluid collector configured to collect fluid expelled from the medical injection device during testing thereof.

In certain configurations, the medical injection device comprises a syringe, and wherein the first portion is a plunger assembly and the second portion is a syringe barrel.

In certain configurations, the testing apparatus comprises a cap retention device configured to receive and retain therein a tip cap of the syringe, the cap retention device secured to the bottom base of the frame.

In certain configurations, the cap retention device comprises a consumable insert including a cavity formed therein sized to receive the tip cap, and wherein an adhesive is provided in the cavity that bonds the tip cap to the cap retention device upon positioning of the tip cap within the cavity.

In certain configurations, the x-ray imaging system is configured to capture two-dimensional dynamic x-ray videos of the medical injection device during testing thereof.

Also provided herein is a method for testing a medical injection device using the system including a testing apparatus and x-ray imaging system, as previously described. The method includes supplying a medical injection device to the testing apparatus and performing a testing of the medical injection device by operating the actuator to move the holder and, in turn, the second portion of the medical injection device, and acquiring x-ray images of the syringe during movement of the second portion of the medical injection device.

In certain configurations, the medical injection device comprises a syringe and performing the testing comprises performing a gliding test where a syringe barrel of the syringe is moved relative to a plunger assembly of the syringe, such that a stopper of the plunger assembly slides within the syringe barrel, with x-ray images acquired of the interaction between the stopper and the syringe barrel.

In certain configurations, the medical injection device comprises a syringe and performing the testing comprises performing a pull-out force test where a tip cap of the syringe is pulled off from a syringe barrel of the syringe, with x-ray images acquired of the removal of the tip cap from the syringe barrel.

### BRIEF DESCRIPTION OF THE DRAWINGS

FIG. 1 is a perspective view of a syringe usable with embodiments of the disclosure;
FIG. 2 is an exploded view of the syringe of FIG. 1;
FIG. 3 is a block schematic diagram of a system for testing of a medical injection device, such as the syringe of FIG. 1, according to a non-limiting embodiment described herein;
FIG. 4 is a front perspective view of a testing apparatus included in the system of FIG. 3, according to a non-limiting embodiment described herein;
FIG. 5 is a rear perspective view of the testing apparatus of FIG. 3;
FIG. 6 is a front elevational view of the testing apparatus of FIG. 3;
FIG. 7 is a rear elevational view of the testing apparatus of FIG. 3;
FIG. 8 is a front perspective view of a testing apparatus included in the system of FIG. 3, according to another non-limiting embodiment described herein; and
FIG. 9 is a detailed view of area A of FIG. 8.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

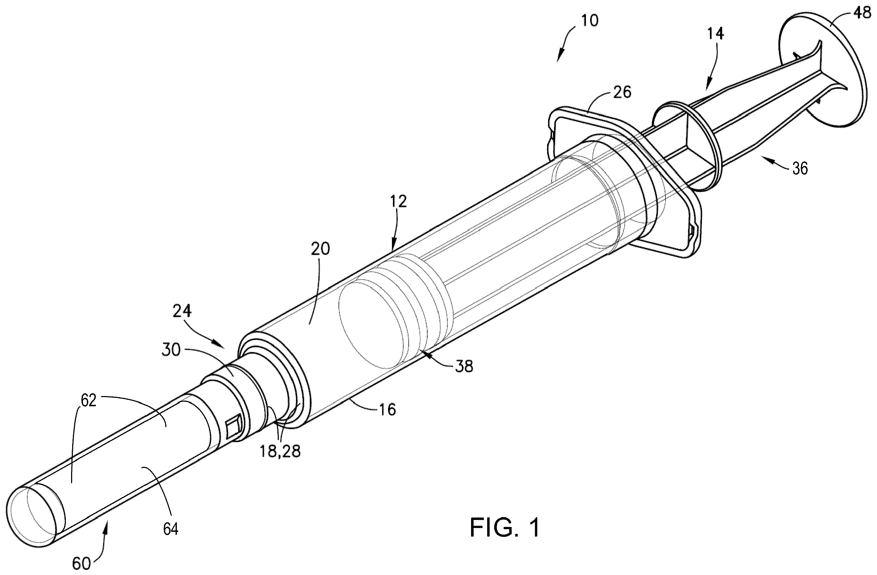

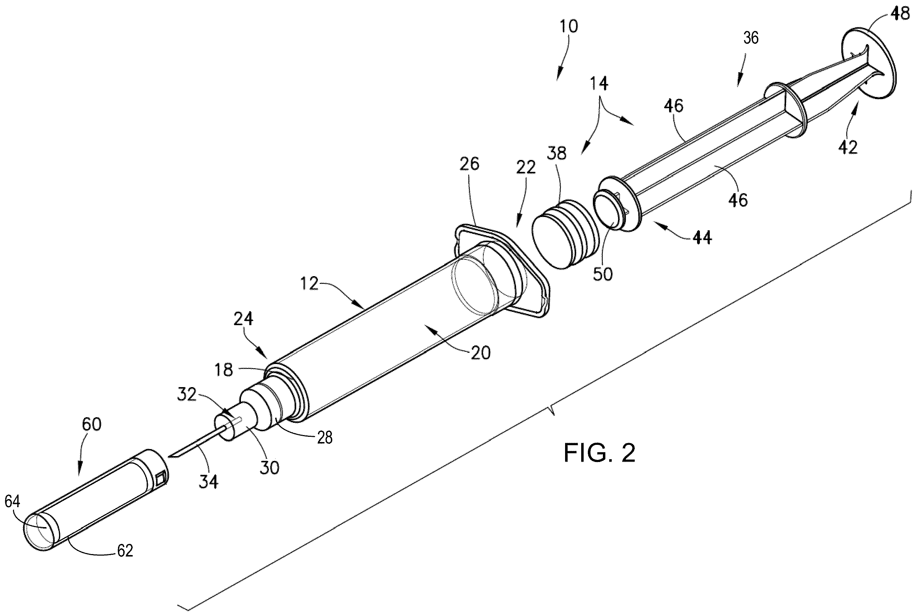

Referring to FIGS. 1 and 2, shown is a non-limiting embodiment of a medical injection device 10, such as a syringe or prefilled syringe for example, with which aspects or embodiments of the disclosure may be implemented. While the medical injection device is shown and described hereafter as a syringe 10, it is recognized that other medical injection devices (e.g., may incorporate aspects of the disclosure, as described in detail here below.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. In some embodiments, a needle 34 is attached to the hub portion 30 within channel 32, such as by being glued or otherwise secured to the hub portion 30. In other embodiments, it is recognized that - rather than needle 34 - syringe 10 could instead include a luer connection formed at the distal end 24 of syringe barrel 12 that enables the syringe 10 to be directly coupled to a luer connection of an associated medical device or component, to provide for the direct transfer of fluids therebetween.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, a flanged extension member 50 (e.g., disc-shaped flange) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper, with the protrusion and receptacle engaging via threading, for example.

The stopper 38 of plunger assembly 14 is positioned at the plunger distal end 44 so as to be movable along with the plunger 36 within the chamber 20 of syringe barrel 12. The stopper 38 may be made from a material that is different from the material of the plunger 36 and that is capable of forming a tight seal with the syringe barrel 12 as it is advanced therethrough. In some embodiments, the stopper 38 includes a receptacle (not shown) formed therein that is sized and configured to receive the flanged extension member 50 of plunger 36, with the flanged extension member 50 coupling with the receptacle via a press fit connection, for example, to secure the stopper 38 to the plunger 36, although it can be appreciated that the stopper 38 and plunger distal end 44 can be secured by other techniques known in the art.

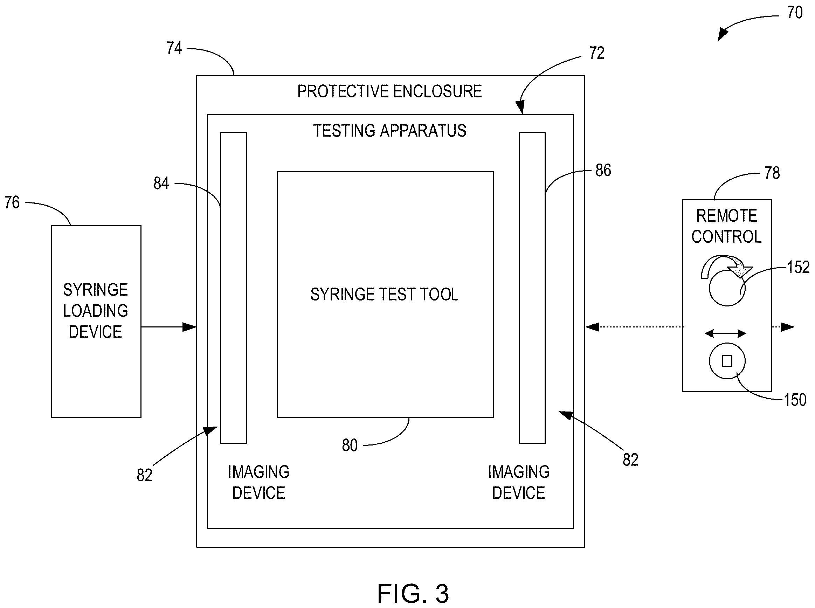

As shown in FIGS. 1 and 2, and now also in FIG. 3, syringe 10 further includes a tip cap 60 that may be coupled to the hub portion 30 of syringe barrel 12. While shown in FIGS. 1 and 2 as a needle shield that is used to protect the needle 34, tip cap 60 could instead be configured as a cap that covers a luer connection. In embodiments where the tip cap 60 is configured as a needle shield, the tip cap 60 may be composed of a rigid outer casing 62 that provides structure to the tip cap 60 and a compliant inner casing 64 that surrounds the needle 34 (when needle shield 60 is coupled to syringe barrel 12). In various embodiments, the outer casing 62 may be made of a rigid material, while the inner casing 64 is made of a deformable material (e.g., rubber). As known in the art, the tip cap 60 may be removed from the hub portion 30 of syringe barrel 12 by application of a pull-out force to tip cap 60 in a direction away from the syringe barrel 12 - with the pull-out force being the force required to remove the needle shield from the hub portion 30 and from needle 34. According to embodiments, the formulation of the soft component of the needle shield and/or the amount and location of contact between the soft component and the syringe barrel can contribute to the amount of pull-out force required to remove the needle shield.

According to aspects of the disclosure, a system is provided by which testing of a medical injection device, such as syringe 10, may be performed. The system allows for testing of the syringe regarding the interaction of components of the syringe during use thereof. As one example, testing may be performed on movement of the stopper within the syringe barrel during actuation/movement of the plunger assembly, to analyze deformation of the stopper, characteristics/reaction of the lubricant layer, or the like. As another example, testing may be performed on removal of the tip cap reacts from the syringe, to analyze deformation of the tip cap, breaking of a frangible feature on the tip cap, or a pull-out force required to remove the tip cap. The testing of the syringe may include acquiring a series of images of the syringe during movement and/or actuation of components thereof, which may include the capturing of video images and/or x-ray images of the syringe.

Referring now to FIG. 3, a block diagram of a system 70 is provided by which testing of syringe 10 may be performed, according to a non-limiting embodiment. The system 70 includes a testing apparatus 72 by which testing of the syringe 10 may be performed, an enclosure 74 within which the testing apparatus 72 is contained, a loading device 76 for providing/loading the syringe 10 into the testing apparatus 72, and a control device 78 that provides for remote operation of the testing apparatus 72 ("remote control 78"). The testing apparatus 72 may further include tooling 80 that provides for retention and actuation/movement of the syringe 10, along with an imaging device 82 that allows for the capture of images or video of the syringe 10 during testing.

According to one aspect of the disclosure, the imaging device 82 included in testing apparatus 72 is an x-ray imaging device that provides for the capture of two-dimensional dynamic x-ray videos of the syringe 10 during testing thereof. The x-ray imaging device 82 may include an arrangement of an x-ray source 84 and x-ray detector 86 that provide for the capture of such two-dimensional dynamic x-ray videos, with the x-ray source 84 and x-ray detector 86 positioned appropriately relative to tooling 80 to enable capturing of such x-ray videos during testing. The x-ray imaging device 82 (along with tooling 80) is contained within enclosure 74, which according to embodiments may comprise a leaded enclosure that prevents a user of system 70 from being exposed to x-ray radiation. In some embodiments, imaging device 82 may be configured to acquire other types of images or videos (other than x-ray images/videos), with a high-resolution video recorder being a non-limiting example.

In some embodiments, the syringe loading device 76 may comprise a robotic arm or device that provides for automated loading of the syringe 10 into testing apparatus 72. The loading device 76 operates to provide a syringe 10 through an opening of enclosure 74 and to the testing apparatus 72 (i.e., to tooling 80), to enable testing of the syringe 10.

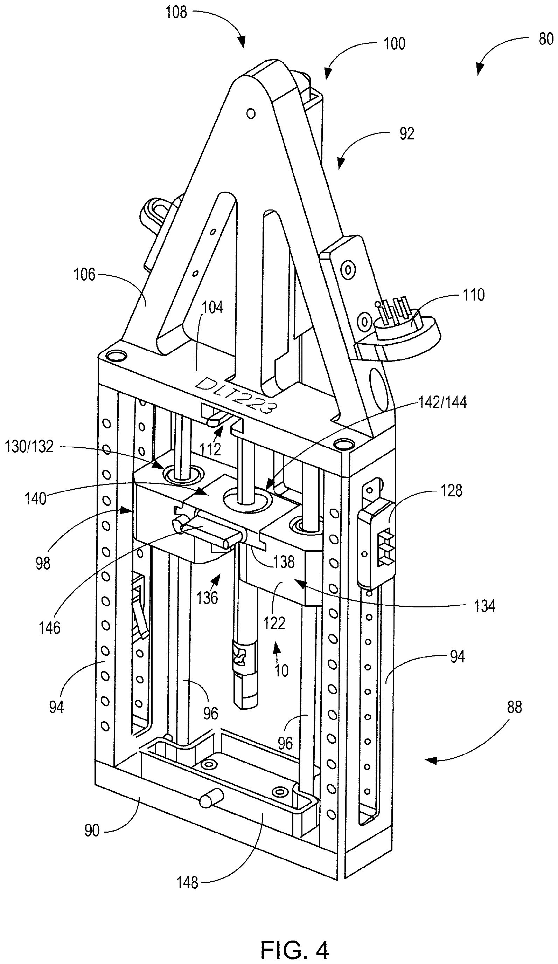

Referring now to FIGS. 4-7, the tooling 80 of testing apparatus 72 is shown in further detail, according to an embodiment of the disclosure. The tooling 80 includes a frame 88 having a bottom base 90, a top support 92 spaced vertically from the bottom base 90, and side track members 94 extending between the top support 92 and the bottom base 90. The tooling 80 also includes guide rails 96 extending vertically between the top support 92 and the bottom base 90, a (syringe) holder 98 mounted on the guide rails 96 and movable therealong relative to the frame 88, and an actuator 100 coupled to the frame 88 and configured to move the holder 98 along the guide rails 96. According to one aspect of the disclosure, components of the tooling 80 are formed of a non-metallic material that is suitable for use with the imaging device 82 of testing apparatus 72, i.e., compatible with an x-ray imaging device. Thus, in some embodiments, the tooling 80 may be formed of a polymeric material or other suitable composite material that does not interfere with the acquisition of x-ray images of the syringe 10 during testing.

As shown in FIGS. 4-7, the frame 88 of tooling 80 is configured as a generally open frame 88 that provides for visibility of the syringe 10 when positioned in/on the tooling 80. The bottom base 90 may comprises a generally flat or planar base, with the side track members 94 positioned on opposing sides of the base 90 and the top support 92 positioned above the side track members 94 - with the frame 88 thus forming a window-type opening 102 within which the guide rails 96 and holder 98 are arranged. The window-type opening 102 defined by the frame 88 allows for the acquisition of images of the syringe 10 (by imaging device 82) during testing without the frame 88 interfering with such image acquisition, such as by enabling an x-ray source 84 and detector 86 to be positioned at opposing front and back sides of the tooling 80 without any portion of the tooling 88 being positioned between the syringe 10 and the source 84 and/or detector 86.

In some embodiments, the top support 92 of frame 88 may be configured as a triangular-shaped member having a bottom leg 104 and side legs 106. The bottom leg 104 of top support 92 is joined to the top end of the side track members 94 and is arranged in parallel with the bottom base 90 of frame 88, with the side legs 106 being angled upwardly from the bottom leg 104 and meeting at an upper end or point 108 of top support 92. The side legs 106 of top support 92 may enable connections to be made to the tooling 80, such as by including a power plug 110 on one of the side legs 106 by which power may be supplied to tooling 80 (i.e., to actuator 100). The bottom leg 104 of top support 92 may be configured to include a slot or receptacle 112 on a front surface thereof that is configured to receive and retain a portion of the syringe 10. In particular, the thumb press 48 at the proximal end 42 of plunger rod 36 may be positioned within the slot 112 in bottom leg 104, such that the plunger rod 36 may remain stationary during actuation and testing of the syringe 10.

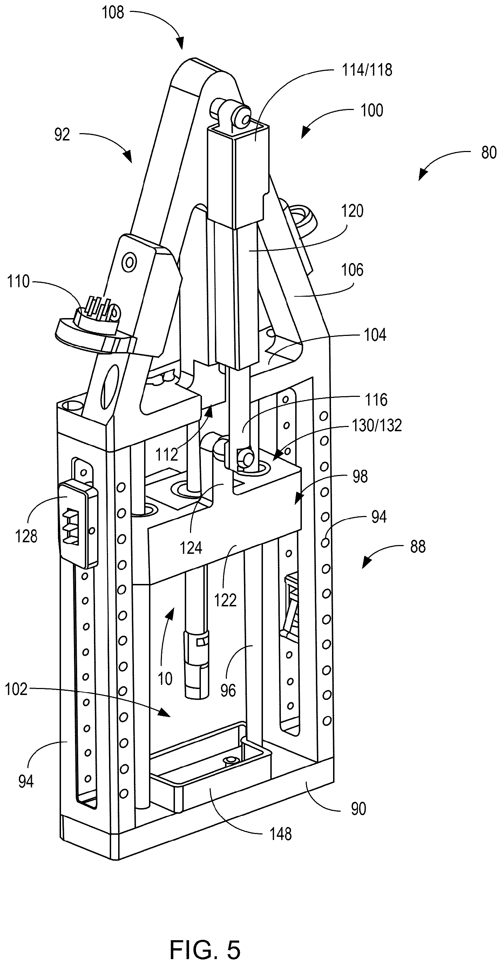

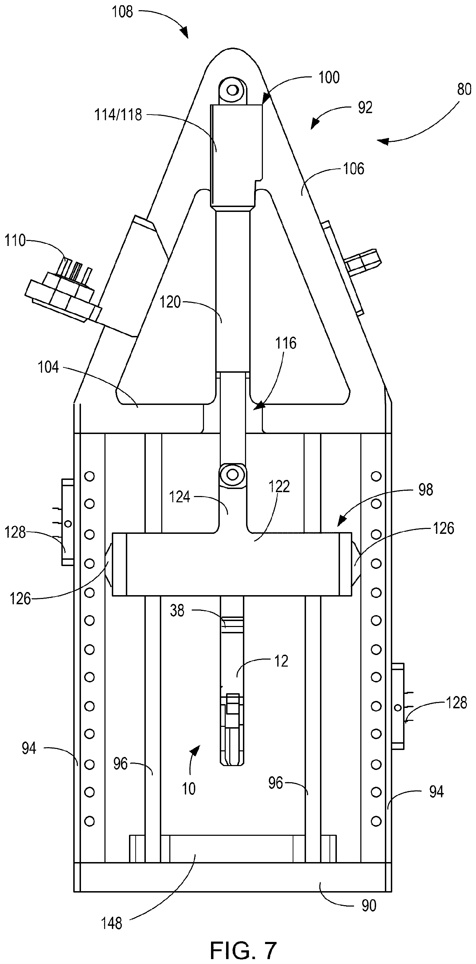

As shown best in FIGS. 5 and 7, actuator 100 is secured to the top support 92 of frame 88, as well as to the holder 98. The actuator 100 may be any type of suitable actuator by which linear movement of the holder 98 may be performed, with the actuator 100 configured as an electrical or hydraulic actuator, as non-limiting examples. In an exemplary embodiment, actuator 100 includes a servo motor 114 that provides for precise control of the linear position of the holder 98, and also (optionally) the velocity, and acceleration of the holder 98 - with the servo motor 114 controlling a positioning/length of an extension rod 116 coupled between the motor and the holder 98. The servo motor 114 is a closed-loop servo-mechanism that uses position feedback to control its motion and the positioning of holder 98. In one example, the servo motor 114 is paired with a position encoder such as a potentiometer 118 to provide position (and, optionally, speed) feedback, for controlling operation thereof. The servo motor 114 may be attached to the frame 88 at the upper end or point 108 of the top support 92, with the extension rod 116 positioned below servo motor 114 within a sleeve 120 and being moved into and out of the sleeve 120 in the vertical direction. A bottom end of extension rod 116 is coupled to holder 98, such that extension and/or retraction of the extension rod 116 out from or back into sleeve 120 causes a vertical movement of the holder 98 and the portion of the syringe 10 retained thereby.

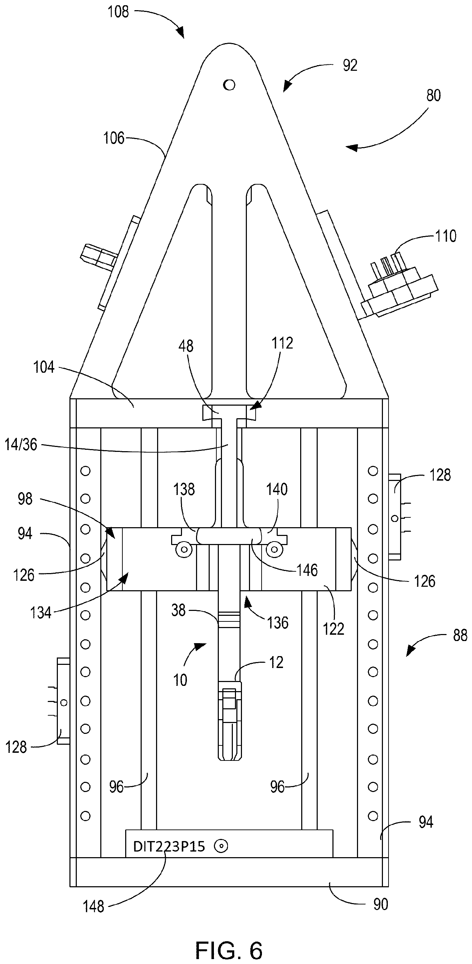

As previously indicated, the holder 98 of testing apparatus 72 is movable along guide rails 96 responsive to operation of the actuator 100. The holder 98 includes a main body 122, a protrusion 124 extending up from the main body 122 (to which extension rod 116 of actuator 100 is secured), and a pair of rollers 126 (FIG. 6) positioned on opposing ends of the main body 122. The rollers 126 are configured to engage the side track members 94 of frame 88 to as to provide for movement of the holder 98 along the side track members 94. In some embodiments, the rollers 126 may include a circumferential groove or protrusion formed thereon that mates and engages with a corresponding track formed on side track members 94, to provide for a smooth translation of the holder 98 along the side track members 94. In some embodiments, upper and lower stops 128 may be provided on the side track members 94 to limit the range of movement of the holder 98 relative to the frame 88. The stops 128 may be affixed to the side track members 94 at desired locations, with the stops 128 functioning to contact the rollers 126 of holder 98 and prevent further movement thereof along the side track members 94. The stops 128 may be affixed to the side track members 94 at desired locations thereon to set the range of movement of the holder 98, with it recognized that the positioning of the stops 128 (and the desired range of movement of the holder 98) may be determined by the type/size of the syringe 10 being tested and/or the type of testing being performed by system 70.

The main body 122 of holder 98 includes a pair of channels 130 formed therein extending from a top end thereof and through the main body 122 to a bottom end thereof, with the channels 130 positioned to receive the guide rails 96 of tooling 80 therein. In some embodiments, bushings 132 may be included within the channels 130 to limit abrasion/friction between the holder 98 and the guide rails 96. The channels 130 (and bushings 132) are configured so as to enable the main body 122 to slide along the guide rails 96 with minimal friction, while providing for stability of the holder 98 on the guide rails 96 and preventing wobbling or other horizontal movement of the main body 122 relative to the guide rails 96.

The main body 122 of holder 98 also includes therein a syringe retention area 132 that is centered on the main body 122 and aligned with the extension rod 116 of actuator 100. The retention area 132 is formed in a front surface 134 of the main body 122 and includes a central receptacle 136 extending vertically through the main body 122 and an upper slot 138 arranged generally orthogonal to the central receptacle 136. The central receptacle 136 is configured to provide for positioning of the barrel 12 of syringe 10 therein, with the syringe barrel 12 extending out from the bottom of main body 122 when positioned within the central receptacle 136. The upper slot 138 formed in the main body 122 is configured to receive a removable holding piece 140 of holder 98 that retains the syringe barrel 12 therein, with grooves on opposing sides of the upper slot 138 configured to retain flanges of the holding piece 140 therein to secure the holding piece 140 to the main body 122. The holding piece 140 includes an opening 142 formed therethrough that is aligned with the central receptacle 136, with the opening 142 having a diameter that is about equal to a diameter of the barrel 12 of syringe 10 - such that the barrel 12 may be snugly received within opening 142. The holding piece 140 also includes a seat 144 formed about the opening 142 on an upper surface thereof, with the seat 144 sized to receive the flange 26 formed at the proximal end 22 of the syringe barrel 12. It is recognized that the removable holding piece 140 may be customized to receive a particular size syringe (barrel) that is to be tested by the testing apparatus 72, with the size of the opening 142 and seat 144 varying depending on the syringe 10 being tested (e.g., a 10mL syringe vs. a 1-3mL syringe). The holding piece 140 may be selectively swapped out for another holding piece 140 when it is desired to perform testing on another size syringe 10, so as to provide flexibility/adaptability in the testing apparatus 72 for testing a large variety of syringes and other medical injection devices. To assist in removal of the holding piece 140, a front flange 146 may be provided on a front end/edge of the holding piece 140 - with the front flange 146 allowing for the holding piece 140 to be gripped and pulled out from the upper slot 138 of main body 122.

According to aspects of the disclosure, the testing apparatus 72 may be employed to perform an activation and gliding test where the plunger assembly 14 is moved relative to the syringe barrel 12, such that the stopper 38 slides within the syringe barrel 12 and the interaction therebetween can be tested and observed. With the holder 98 and frame 88 configured as described above, a syringe 10 positioned in the testing apparatus 72 will have its barrel 12 secured within the holder 98 (with the holding piece 140 receiving the syringe barrel 12 therein) and its plunger assembly 14 secured to the frame 88 (with the slot 112 in top support 92 receiving the thumb press 48 of plunger rod 36 therein). By operation of the of the actuator 100, the syringe barrel 12 and plunger assembly 14 may be moved relative to one another, such as by repositioning/moving the syringe barrel 12 upwards or downwards with the plunger rod 36 retained in a fixed position. During movement of the syringe barrel 12 relative to the plunger assembly 14 (and stopper 38), the imaging device 82 can acquire a plurality of images or dynamic video of the movement, such as two-dimensional dynamic x-ray videos of the syringe 10 as the stopper 38 is moved within the syringe barrel 12.

In some embodiments, where the syringe 10 being tested is provided as a prefilled syringe that includes a medicament or other fluid within the chamber 20 of the syringe barrel 12, a fluid collector 148 may be provided as part of the testing apparatus 72 to catch/collect fluid dispensed from the syringe 10 during testing. The fluid collector 148 may have a bowl-like construction and may be positioned on a top surface of bottom base 90 - with the fluid collector 148 centered on the bottom base 90 and generally extending between the guide rails 96 of the testing apparatus 72. As the plunger assembly 14 and stopper 38 are moved further into the chamber of syringe barrel 12 during testing (responsive to movement of holder 98 via actuator 100), fluid is dispensed out of the chamber 20 of syringe barrel 12, with the fluid being caught by the fluid collector 148 and held thereby, so as to prevent spillage of the fluid into other areas of the testing apparatus 72 and/or enclosure 74.

As indicated above, system 70 includes a control device 78 (i.e., remote control 78) that provides for remote operation of the testing apparatus 72. The remote control 78 may be configured to send commands to the actuator 100 to control operation thereof, such as by controlling operation of the servo motor 114 to effect a linear translation of the holder 98 and thereby actuate the syringe 10. As shown in FIG. 3, the remote control 78 may include a plurality of input devices 150, 152 thereon that may be used to control operation of the actuator 100 and/or other components of the system 70 (e.g., syringe loading device 76). In one embodiment, the input devices 150, 152 may include a toggle switch 150 and a rotatable knob 152 that control the actuator 100. The toggle switch 150 may control upward and downward movement of the holder 98 to selectively actuate the syringe 10, with movement of the toggle switch 150 in a first direction causing the actuator 100 to move the holder 98 of testing apparatus 72 in a downward direction and movement of the toggle switch 150 in a second direction causing the actuator 100 to move the holder 98 of testing apparatus 72 in an upward direction. The rotatable knob 152 may control a velocity at which the actuator 100 moves the holder 98 in the upward and downward direction, with rotation of the knob in a first direction causing the actuator 100 to increase the velocity at which the holder 98 is translated and rotation of the knob in a second direction causing the actuator 100 to decrease the velocity at which the holder 98 is translated.

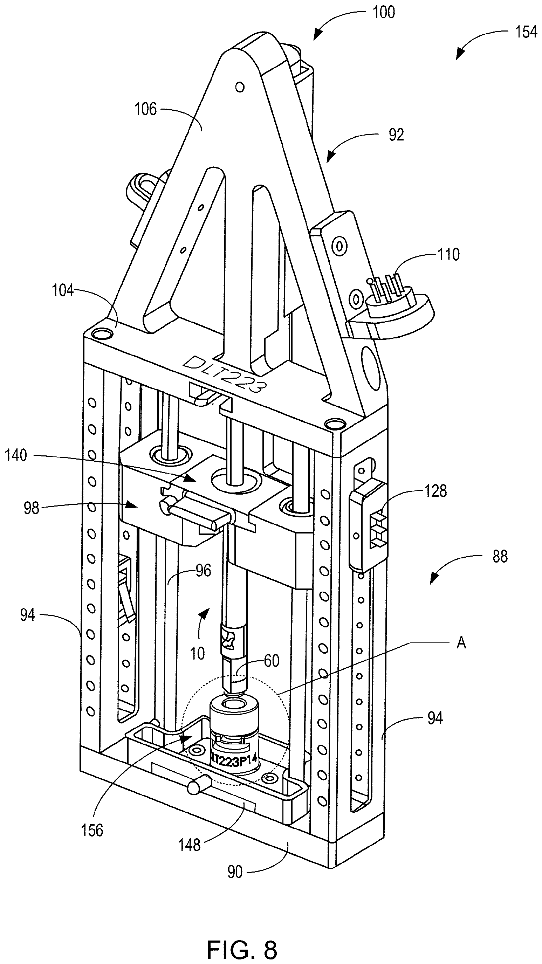

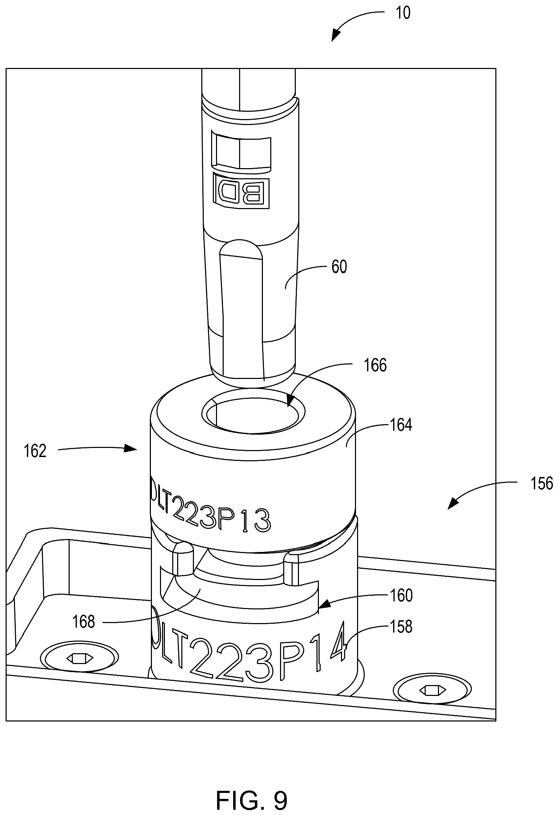

Referring now to FIGS. 8 and 9, a tooling 154 for testing apparatus 72 is shown according to another embodiment of the disclosure. The tooling 154 is substantially similar to the tooling 80 of FIGS. 4-7, except that the tooling 154 is specifically configured to perform a testing directed toward the pullout force (POF) required for removing the tip cap 60 from the syringe 10. In order to enable performing of a POF test on the syringe 10, the tooling 154 further includes a cap retention device 156 that is able to secure the tip cap 60 of syringe 10 therein - with the actuator 100 and holder 98 then functioning to pull the syringe barrel 12 away from the tip cap 60 while it is secured in the cap retention device 156, in order to cause the tip cap 60 to separate from the syringe barrel 12.

As shown in FIGS. 8 and 9, the cap retention device 156 may include a retention fixture 158 that is secured to the bottom base 90 of frame 88, such as via appropriate fasteners. In some embodiments, the retention fixture 158 may be positioned within fluid collector 148, which may be left in place on bottom base 90, while in other embodiments the retention fixture 158 may be secured directly or indirectly (via another fixture plate) to the bottom base 90. The retention fixture 158 may comprise a generally cylindrical member that includes a slot 160 formed toward an upper end thereof, with the slot 160formed through/about a portion of a circumference of the cylindrical member.

According to aspects of the disclosure, the retention fixture 158 (i.e., the slot 160 formed therein) receives a consumable insert 162 that is configured to secure the tip cap 60 therein. As best shown in FIG. 9, the consumable insert 162 may comprise a cylindrical top portion 164 having an opening or cavity 166 formed in a top surface thereof, along with a bottom portion 168 that is in the form of an annular flange that is separated vertically from the cylindrical top portion 164 by a gap. The bottom annular flange 168 is sized and configured to engage with the slot 160 formed in the upper end of the retention fixture 158, such that the consumable insert 162 may be secured to the retention fixture 158 and to the overall frame 88 of tooling 154. The cavity 166 formed in the cylindrical top portion 164 is sized and configured to receive the tip cap 60 of syringe 10 therein - with the cavity 166 thus having a diameter similar to that of tip cap 60. The cavity 166 includes therein an adhesive that may be used to secure the tip cap 60 to the cap retention device 156 upon positioning of the tip cap 60 in the cavity 166. The adhesive may be a quick drying or quick bonding adhesive that is able to bond with the tip cap 60 upon placement of the tip cap 60 within cavity 166, thereby securing the tip cap 60 to the consumable insert 162.

In performing the POF test, the testing apparatus 72 may be initially operated such that actuator 100 causes the holder 98 to move downwardly, thereby moving the syringe barrel 12 and tip cap 60 downward until the tip cap 60 engages the cap retention device 156. Specifically, the syringe barrel 12 and tip cap 60 are moved downward until the tip cap 60 is positioned within the cavity 166 of consumable insert 162. Upon the adhesive within cavity 166 bonding with the tip cap 60 to secure the tip cap 60 to the consumable insert 162, the testing apparatus 72 may then be operated such that actuator 100 causes the holder 98 to move upwardly, thereby applying a pulling force to the syringe barrel 12 in a direction away from the cap retention device 156. With the syringe barrel 12 being pulled away from the cap retention device 156 (and the tip cap 60 retained therein), the tip cap 60 is caused to separate from the syringe barrel 12. During this separation of the syringe barrel 12 from the tip cap 60, the imaging device 82 can acquire a plurality of images or dynamic video of the movement, such as two-dimensional dynamic x-ray videos of the syringe as the tip cap 60 is removed from the syringe barrel 12. Accordingly, POF testing may be performed via the tooling 154 illustrated in FIGS. 8 and 9.

Beneficially, embodiments of the invention thus provide a system for testing a medical injection device (e.g., syringe). The system includes a testing apparatus and associated tooling that provides for image acquisition of a syringe during testing, including x-ray images and/or other video images. The tooling is multi-functional, so as to enable testing of various types/sizes of syringes, and for the testing of different components of the syringe, such as may be desired for testing associated with movement of the plunger assembly and/or removal of a tip cap. The system provides for remote operation of the testing apparatus, such as may be involved with actuating the syringe during testing.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A system for testing of a medical injection device, the system comprising:
a testing apparatus configured to retain the medical injection device for testing thereof, the testing apparatus including:
an open frame comprising:
a bottom base;
a top support spaced vertically from the bottom base, the top support configured to secure a first portion of the medical injection device; and
side track members extending between the top support and the bottom base;
guide rails extending vertically between the top support and the bottom base;
a holder movable along the guide rails and the side track members relative to the frame, the holder configured to secure a second portion of the medical injection device; and
an actuator coupled to the frame and configured to move the holder in an upward direction and a downward direction along the guide rails, with movement of the holder causing a relative movement between the second portion and the first portion of the medical injection device; and
an x-ray imaging system configured to acquire x-ray images of the medical injection device, so as to capture the relative movement between the second portion and the first portion of the medical injection device.

2. The system of claim 1, further comprising an x-ray proof enclosure positioned about the testing apparatus and the x-ray imaging system.

3. The system of claim 2, further comprising a loading device configured to provide the medical injection device to the testing apparatus, with the loading device transferring the medical injection device to the holder.

4. The system of any of claims 1-3, further comprising a remote control configured to control operation of the actuator.

5. The system of claim 4, wherein the remote control includes:
a first control input to control movement of the holder in the upward direction and the downward direction; and
a second control input to control a speed of the holder as it moves along the guide rails.

6. The system of any of claims 3-5, wherein the actuator comprises:
an outer sleeve;
an extension rod movable within the outer sleeve, with a bottom end of the extension rod joined to the holder; and
a servo motor configured to linearly translate the extension rod into and out from the outer sleeve, to move the holder in the upward direction and the downward direction.

7. The system of any of claims 1-6, wherein the testing apparatus further comprises a pair of stops positioned on the side track members and configured to limit a range of movement of the holder in the upward and downward directions along the side track members.

8. The system of any of claims 1-7, wherein the top support includes a retention slot formed therein by which the first portion of the medical injection device is secured to the top support.

9. The system of any of claims 1-8, wherein the testing apparatus further comprises a fluid collector positioned on the bottom base, the fluid collector configured to collect fluid expelled from the medical injection device during testing thereof.

10. The system of any of claims 1-9, wherein the medical injection device comprises a syringe, and wherein the first portion is a plunger assembly and the second portion is a syringe barrel.

11. The system of claim 10, wherein the testing apparatus further comprises a cap retention device configured to receive and retain therein a tip cap of the syringe, the cap retention device secured to the bottom base of the frame.

12. The system of claim 11, wherein the cap retention device comprises a consumable insert including a cavity formed therein sized to receive the tip cap, and wherein an adhesive is provided in the cavity that bonds the tip cap to the cap retention device upon positioning of the tip cap within the cavity.

13. The system of any of claims 1-12, wherein the x-ray imaging system is configured to capture two-dimensional dynamic x-ray videos of the medical injection device during testing thereof.

14. A method for testing a medical injection device using the system of claim 1, the method comprising:
supplying a medical injection device to the testing apparatus; and
performing a testing of the medical injection device by:
operating the actuator to move the holder and, in turn, the second portion of the medical injection device; and
acquiring x-ray images of the medical injection device during movement of the second portion of the syringe.

15. The method of claim 14, wherein the medical injection device comprises a syringe, and wherein performing the testing comprises performing a gliding test where a syringe barrel of the syringe is moved relative to a plunger assembly of the syringe, such that a stopper of the plunger assembly slides within the syringe barrel, with x-ray images acquired of the interaction between the stopper and the syringe barrel.

16. The method of claim 14, wherein the medical injection device comprises a syringe, and wherein performing the testing comprises performing a pull-out force test where a tip cap of the syringe is pulled off from a syringe barrel of the syringe, with x-ray images acquired of the removal of the tip cap from the syringe barrel.
